# EUROPEAN PATENT SPECIFICATION

(11) **EP 0 858 347 B1**
(45) Date of publication and mention of the grant of the patent: **02.03.2005**
(21) Application number: 96936725.9
(22) Date of filing: 18.10.1996
(51) Int. Cl.: A61M 1/00, B01D 35/00, B01D 35/01, B01D 35/30

(54) **CONTAINER WITH INTEGRAL PUMP PLATEN**
BEHÄLTER MIT INTEGRALER PUMPENDRUCKPLATTE
RECIPIENT AVEC PLATINE DE POMPE MONOBLOC

(30) Priority: 20.10.1995 US 8127 P; 20.10.1995 US 8128 P; 20.10.1995 US 5772 P
(43) Date of publication of application: 19.08.1998
(73) Proprietor: Harvest Technologies Corporation, Norwell, MA 02061 (US)
(72) Inventor: VERKAART, Wesley, H., Norwell, MA 02061 (US)
(74) Representative: Crouch, David John
(86) International application number: PCT/US1996/016770
(87) International publication number: WO 1997/014450

(56) References cited:
- EP-A- 0 254 607
- US-A- 4 135 647
- US-A- 4 275 731
- US-A- 4 681 571
- US-A- 5 000 351
- US-A- 5 087 250
- US-A- 5 330 431
- US-A- 5 372 709
- US-A- 5 458 567
- US-A- 5 512 042

## Description

### TECHNICAL FIELD

This invention relates to the art of articles for the collection and discharge of fluids. In particular, the invention relates to a disposable container for the collection of physiological fluids and transfer of the fluids to another container.

### BACKGROUND

It is known to collect fluids, such as blood, from surgical sites with the use of vacuum pressures. The blood thus collected is often treated to remove air and particulate contaminants. Known filters for physiological fluids typically employ such features as a tubular inlet directed tangentially to a cylindrical inlet chamber to generate centrifugal forces for separating the air from collected liquid and physical filters for further removal of particulate contaminants. These devices, however, do not lend themselves to easy discharge of the collected fluids and do not provide for effective treatment of the collected blood with anticoagulants, and the like.

US-A-4,135,647 discloses a dispensing unit mountable on a can and like container for dispensing a liquid content therefrom. The dispensing unit includes a resilient tube which is passed into the can and has a portion seated on the end wall of the can and terminates in a nozzle. A pumping unit is mounted in overlying relation to the tube portion lying on the end wall and is operable progressively and intermittently to apply pressure against the tube portion to collapse the same in a pumping action. The dispensing unit is battery powered electric motor driven and may include a detachable supporting base carrying a battery charger.

US-A-4,275,731 discloses a canister having suction and fluid receiving ports. A vacuum is applied to the suction port to reduce the pressure in the canister, thus drawing fluid into the canister through the fluid receiving port. The canister is also provided with a pour spout through which collected fluids pass when the canister is emptied.

### SUMMARY OF THE INVENTION

The present invention is directed to apparatus, for collection and discharge of fluids, as set out in the accompanying claim 1. Advantageous preferred features are set out in the sub-claims 2 to 9.

In accordance with the invention, a blood collection chamber includes a vacuum chamber for accumulating a collected fluid, such as blood or other physiological fluids. An integral portion of the container forms at least a portion of a pump for discharging the collected fluid rom the container. The apparatus also includes a discharge port for allowing said fluids to flow out of said chamber. In a preferred embodiment, the chamber is used in a system for vacuum collection of blood and other physiological products from a surgical site.

The container may be divided into upper and lower chambers. The upper chamber is cylindrical and receives the fluid from the surgical site and performs an initial separation of air and debris from the fluid. The initial separation may be accomplished by directing the fluid into the chamber to form a vortex. The fluid is then passed through a filter between the upper and lower chambers for further cleaning. In a preferred embodiment, the vortex is created by introducing the fluids in a direction tangent to the upper chamber. A vacuum port communicates with the lower chamber to create a pressure differential that draws fluids from the surgical site into the upper chamber and then from the upper chamber into the lower chamber with the assistance of gravity.

The upper chamber may be provided with a port for admitting a second fluid to be mixed with the collected fluids. In a preferred embodiment, the second fluid is an anticoagulant. The amount of anticoagulant drawn into the chamber is a function of the level of vacuum pressure. Accordingly, more anticoagulant is drawn in as more fluid is drawn in, which tends to maintain the proportion of the anticoagulant to that of the fluid collected in the container. This obviates the need for a separate pump or metering device for the anticoagulant.

A feature of the invention is the means by which the collected fluids are discharged from the container. Because the container is under negative pressure, the collected fluids must be pumped out of the container. A known type of pump is a roller pump. The bottom of the lower chamber may be formed into a platen for such a roller pump. The outer tube is positioned adjacent the platen such that it will be pushed against the platen by the rollers when the chamber is placed onto the roller. Thus, the roller pump will be automatically loaded with the tubing and will be capable of operation immediately after the chamber is placed on the support containing the roller.

### BRIEF DESCRIPTION OF THE DRAWINGS

Figure 1 is a perspective view of a chamber in accordance with the invention.
Figure 2 is a transverse cross section of the chamber shown in figure 1 and installed on a roller pump base.

### DETAILED DESCRIPTION OF THE PREFERRED EMBODIMENT

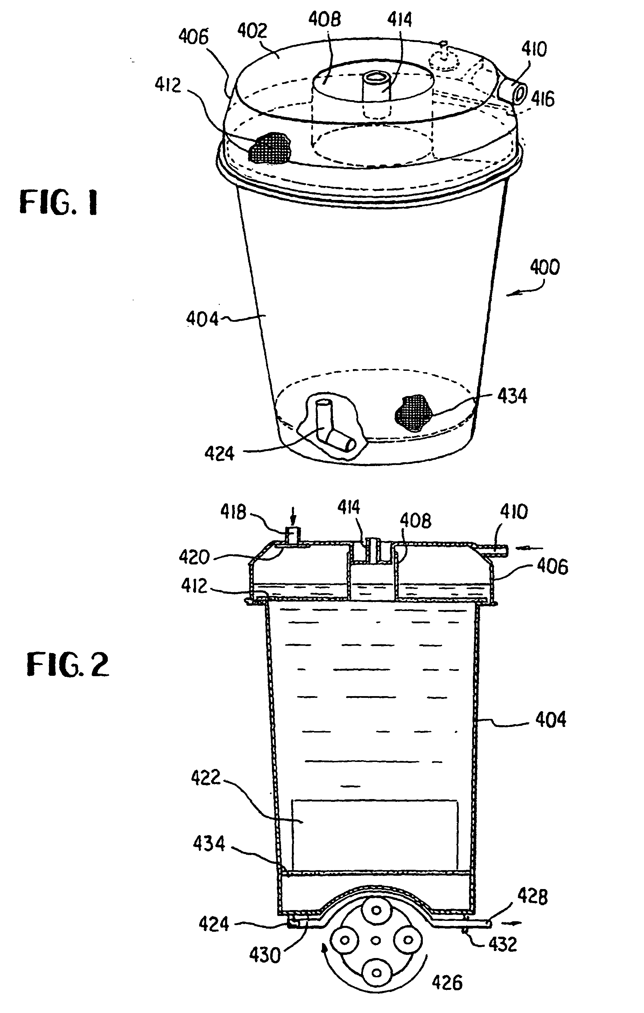

With reference to figure 1, a chamber 400 in accordance with the invention comprises an upper element 402 that is attached to a lower element 404. The upper element includes an outer wall 406 and an inner wall 408, which form a generally cylindrical upper chamber, and a fluid inlet 410. The floor of the upper chamber includes a particulate filter 412 having openings in the range of 400µ to 600µ. A vacuum port 414 communicates with an inner cavity formed by the wall 408, and this cavity is in open communication with the lower chamber formed by the lower element 404.

When vacuum, for example as obtained from a vacuum pump (not shown) is applied to the port 414, air and fluid are drawn into the inlet 410. The inlet is preferably tubular, and the axis of the tube is tangential to the upper chamber. The incoming fluid from the surgical site thus forms a vortex in the upper chamber to begin the separation of air and debris from the liquid. The vortex is terminated by a baffle 416, and the fluid and air are then caused to pass through the filter 412 removing debris from the fluid. The fluid and air pass through filter 412 both by the forces of gravity and by the pressure gradient existing between the upper and lower chambers created by the presence of the filter 412.

A second fluid, preferably an anticoagulant, is introduced to the fluid in the upper chamber through port 418. For example, a bag of anticoagutant may be hung above the container and connected to the port 418 by a flexible tube. The bottom of the port 418 is covered by a porous disk 420, which in turn communicates with the upper chamber. The amount of anticoagulant drawn into the upper chamber will depend in large measure on the level of the vacuum in the upper chamber. Thus, as the vacuum increases, more fluid is drawn through inlet 410, and more anticoagulant is drawn in through port 418. This causes the proportion of anticoagulant in the mixture of collected fluid and anticoagulant to remain constant when the vacuum levels are controlled. Moreover, because the disk presents a relatively large surface area to the upper chamber, the pressure in the port 418 is easily made large enough to draw the desired amount of anticoagulant into the fluid without the use of another mechanical pump for the desired levels of vacuum in the inlet port 410.

In the preferred embodiment, the vacuum pressure applied to the port 414, is controlled whereby the vacuum pressure is increased (e.g., to about -100 mm Hg) only in response to detection of fluids in the collection tube that communicates with the port 410. When the system determines that no fluid is present in the collection tube, as by sensing the pressure drop across a restriction in the collection line, the vacuum pressure is reduced to a much smaller level (e.g., about -20 mm Hg). Thus, when the system is not collecting fluid, little or no anticoagulant is drawn into the chamber due to the reduced vacuum.

The disk 420 is preferably made of a known porous material, such as sintered polyethylene, with pores in the range of from 10µ to 20µ. Alternatively, a mechanical valve may be used separately or in conjunction with the porous disk. The ease of passage of the anticoagulant will depend on such variables as the hydrophobicity of the material, the thickness, and the geometry of the disk, or in the case of the valve, cracking pressure or restriction to flow.

The fluid 422 collected in the chamber will accumulate in the lower chamber. The chamber, however, further includes means for discharging the fluid 422 through a discharge port 424. The discharge means preferably comprises a roller pump, and the pump includes a roller assembly 426 of known construction mounted for rotation about a horizontal axis. A roller pump requires a platen for cooperation with the roller assembly, and this platen in formed into the bottom of the chamber in accordance with the invention. Thus, a flexible outlet line 428 is connected to the discharge port 424 adjacent a recess 430 in the bottom of the chamber, which forms a platen for the roller pump. The outlet line 428 is supported just below the platen 430 by engagement with the port 424 and a clip 432. When the chamber is placed on the roller 426, the line 428 is pushed upward to engage the platen 430 and be compressed between the platen and the rollers. The collected fluids are then discharged by rotation of the roller 426.

If desired, a second filter 434 may be placed above the discharge port to provide additional cleaning of the fluids prior to discharge.

Modifications within the scope of the appended claims will be apparent to those of skill in the art.

## Claims

1. Apparatus for collection and discharge of fluids comprising a chamber (404, 406) for receiving said fluids, a fluid inlet (410) for admitting said fluids to said chamber, a vacuum port (414) in fluid communication with said chamber, and a discharge port (424) separate from said fluid inlet for allowing said fluids to flow out of said chamber, **charactesised by** a flexible outlet line (428) attached to said discharge port for carrying discharged fluids, and a recess (430) in said chamber adjacent said flexible outlet line, said recess forming a platen for engaging said flexible outlet line when said flexible outlet line is pushed into engagement with said recess by rollers of a roller pump to pump said fluids through said discharge port.

2. Apparatus according to claim 1 **characterised in that** said recess is in the bottom of said chamber.

3. Apparatus according to claim 1 **characterised in that** said chamber comprises an upper chamber (402) and a lower chamber (404) in fluid communication with said upper chamber and said fluid inlet is tangentially located in said upper chamber to form a vortex flow in said fluids that are admitted to said upper chamber.

4. Apparatus according to claim 3 **characterised in that** said upper chamber is cylindrical.

5. Apparatus according to claim 3 **characterised in that** said vacuum port (414) is in communication with said lower chamber.

6. Apparatus according to claim 5 **characterised in that** said vacuum port communicates with an inner cavity formed in said upper chamber, said inner cavity being in communication with said lower chamber.

7. Apparatus according to claim 5 **characterised by** a clip (432) for supporting one end of said flexible outlet line.

8. Apparatus according to claim 5 **characterised by** a filter (412) between said upper and lower chambers for removing particulates from said fluids passing from said upper chamber to said lower chamber.

9. Apparatus according to claim 1 **characterised in that** said chamber comprises an upper chamber (402) and a lower chamber (404), said upper chamber is formed between an outer wall and an inner wall, said inner wall forms an inner cavity in communication with said lower chamber, and further **characterised by** a vacuum port connected to said inner cavity for conducting air from said chamber and reducing the pressure in said chamber.

## Patentansprüche

1. Vorrichtung zum Sammeln und Ausgießen von Flüssigkeiten, umfassend eine Kammer (404, 406) zur Aufnahme dieser Flüssigkeiten, einen Flüssigkeitseinlass (410) zum Einführen der Flüssigkeit in die Kammer, einen Vakuumanschluss (414), der in Flüssigkeitsverbindung mit der Kammer steht, und eine vom Flüssigkeitseinlass getrennte Auslassöffnung (424), die es ermöglicht, dass die Flüssigkeiten aus der Kammer herausfließen, **gekennzeichnet durch** eine biegsame Auslassleitung (428), die an der Auslassöffnung befestigt ist, um abgelassene Flüssigkeit zu befördern, und eine Ausnehmung (430) in der genannten Kammer in Nachbarschaft zur biegsamen Auslassleitung, wobei die genannte Ausnehmung eine Andruckplatte zur Anlage für die biegsame Auslassleitung bildet, wenn die genannte biegsame Auslassleitung **durch** Rollen einer Rollerpumpe gegen die genannte Ausnehmung gedrückt wird, um die Flüssigkeiten **durch** die Auslassöffnung zu pumpen.

2. Vorrichtung nach Anspruchs 1, **dadurch gekennzeichnet, dass** sich die Ausnehmung im Boden der Kammer befindet.

3. Vorrichtung nach Anspruch 1, **dadurch gekennzeichnet, dass** die genannte Kammer eine obere Kammer (402) und, in Flüssigkeits-Verbindung mit der genannten oberen Kammer, eine untere Kammer (404) besitzt und der genannte Flüssigkeitseinlass tangential in der oberen Kammer angeordnet ist, um einen Wirbelfluss in den Flüssigkeiten zu erzeugen, die der genannten oberen Kammer zugeführt werden.

4. Vorrichtung nach Anspruch 3, **dadurch gekennzeichnet, dass** die obere Kammer zylindrisch ist.

5. Vorrichtung gemäß Anspruch 4, **dadurch gekennzeichnet, dass** sich der Vakuumanschluss (414) in Verbindung mit der unteren Kammer befindet.

6. Vorrichtung gemäß Anspruch 5, **dadurch gekennzeichnet, dass** der genannte Vakuumanschluss mit einem in der genannten oberen Kammer ausgebildeten Hohlraum verbunden ist, wobei der innere Hohlraum eine Verbindung zur unteren Kammer besitzt.

7. Vorrichtung nach Anspruch 5, **gekennzeichnet durch** einen Halter (432) zum Halten eines Endes der genannten biegsamen Auslassleitung.

8. Vorrichtung gemäß Anspruch 5, **gekennzeichnet durch** einen Filter (412) zwischen der oberen und der unteren Kammer, um feste Teilchen aus den Flüssigkeiten zu entfernen, die von der oberen Kammer in die untere Kammer treten.

9. Vorrichtung gemäß Anspruch 1, **dadurch gekennzeichnet, dass** die genannte Kammer eine obere Kammer (402) und eine untere Kammer (404) aufweist, wobei die obere Kammer zwischen einer Außenwand und einer Innenwand ausgebildet ist und die Innenwand einen inneren Hohlraum bildet, der sich in Verbindung mit der unteren Kammer befindet, und weiterhin **gekennzeichnet durch** einen Vakuumanschluss, der in Verbindung mit dem inneren Hohlraum steht, um Luft aus dieser Kammer zu ziehen und den Druck in dieser Kammer zu verringern.

## Revendications

1. Appareil permettant de récupérer et d'évacuer des fluides, comprenant une chambre(404, 406) pour recevoir lesdits fluides, une entrée de fluide (410) pour admettre lesdits fluides jusqu'à ladite chambre, un orifice sous vide d'air (414) en communication fluide avec ladite chambre, et un orifice d'évacuation (424) séparé de ladite entrée de fluide pour permettre auxdits fluides de s'écouler hors de ladite chambre, **caractérisé par** une conduite de sortie souple (428) attachée audit orifice d'évacuation afin de transporter les fluides évacués, et un enfoncement (430) dans ladite chambre adjacent à ladite conduite de sortie souple, ledit enfoncement formant une platine pour être en contact avec ladite conduite de sortie souple lorsque ladite conduite de sortie souple est poussée au contact dudit enfoncement par des rouleaux d'une pompe à rouleaux afin de pomper lesdits fluides à travers ledit orifice d'évacuation.

2. Appareil selon la revendication 1, **caractérisé en ce que** ledit enfoncement est situé dans la partie inférieure de ladite chambre.

3. Appareil selon la revendication 1, **caractérisé en ce que** ladite chambre comprend une chambre supérieure (402) et une chambre inférieure (404) en communication fluide avec ladite chambre supérieure, et ladite entrée de fluide est située de manière tangentielle dans ladite chambre supérieure afin de former un écoulement tourbillonnaire au sein desdits fluides qui sont admis dans ladite chambre supérieure.

4. Appareil selon la revendication 3, **caractérisé en ce que** ladite chambre supérieure est cylindrique.

5. Appareil selon la revendication 3, **caractérisé en ce que** ledit orifice sous vide d'air (414) est en communication avec ladite chambre inférieure.

6. Appareil selon la revendication 5, **caractérisé en ce que** ledit orifice sous vide d'air communique avec une cavité interne formée dans ladite chambre supérieure, ladite cavité interne étant en communication avec ladite chambre inférieure.

7. Appareil selon la revendication 5, **caractérisé par** une attache (432) permettant de soutenir une extrémité de ladite conduite de sortie souple.

8. Appareil selon la revendication 5, **caractérisé par** un filtre (412) entre lesdites chambres supérieure et inférieure afin de retirer des particules desdits fluides passant de ladite chambre supérieure à ladite chambre inférieure.

9. Appareil selon la revendication 1, **caractérisé en ce que** ladite chambre comprend une chambre supérieure (402) et une chambre inférieure (404), ladite chambre supérieure est formée entre une paroi externe et une paroi interne, ladite paroi interne forme une cavité interne en communication avec ladite chambre inférieure, et **caractérisé en outre par** un orifice sous vide d'air raccordé à ladite cavité interne pour conduire de l'air depuis ladite chambre et pour réduire la pression dans ladite chambre.
